# EUROPEAN PATENT SPECIFICATION

(11) **EP 3 161 488 B1**
(45) Date of publication and mention of the grant of the patent: **22.05.2019**
(21) Application number: 15739524.5
(22) Date of filing: 23.06.2015
(51) Int. Cl.: G01N 33/574, G01N 33/576

(54) **METHOD FOR DIAGNOSING THE RISK OF PRENEOPLASTIC AND NEOPLASTIC LIVER DISEASE IN SUBJECTS AFFECTED BY HEPATITIS**
VERFAHREN ZUR DIAGNOSE DES RISIKOS AN PRÄNEOPLASTISCHER UND NEOPLASTISCHER LEBERERKRANKUNG BEI PERSONEN MIT HEPATITIS
MÉTHODE POUR DIAGNOSTIQUER LE RISQUE DE MALADIE DU FOIE PRÉNÉOPLASIQUE ET NÉOPLASIQUE CHEZ DES SUJETS AFFECTÉS PAR L'HÉPATITE

(30) Priority: 25.06.2014 IT PZ20140005
(43) Date of publication of application: 03.05.2017
(73) Proprietor: Università degli Studi della Basilicata, 85100 Potenza (IT)
(72) Inventor: OSTUNI, Angela, I-85100 Potenza (IT); BISACCIA, Faustino, I-85010 Vaglio di Basilicata (IT)
(74) Representative: Zaccaro, Elisabetta
(86) International application number: PCT/EP2015/064043
(87) International publication number: WO 2015/197579

(56) References cited:
- CN-A- 103 543 265
- Asus: "Angela Ostuni nata a", , 27 September 2013 (2013-09-27), XP055174215, Retrieved from the Internet: URL:http://scienze.unibas.it/site/home/dip artimento/personale/docente/documento10013 21.html [retrieved on 2015-03-05]
- OSTUNI A ET AL: "The hepatitis B x antigen anti-apoptotic effector URG7 is localized to the endoplasmic reticulum membrane", FEBS LETTERS, vol. 587, no. 18, 31 July 2013 (2013-07-31), pages 3058-3062, XP028708826, ISSN: 0014-5793, DOI: 10.1016/J.FEBSLET.2013.07.042
- H.-W. L. HANN ET AL: "Preneoplastic Markers of Hepatitis B Virus-Associated Hepatocellular Carcinoma", CANCER RESEARCH, vol. 64, no. 20, 15 October 2004 (2004-10-15), pages 7329-7335, XP055174287, ISSN: 0008-5472, DOI: 10.1158/0008-5472.CAN-04-1095
- Tong Gd ET AL: "[Preneoplastic markers of hepatitis B virus-associated hepatocellul... - PubMed - NCBI", , 1 November 2007 (2007-11-01), XP055174335, Retrieved from the Internet: URL:http://www.ncbi.nlm.nih.gov/pubmed/180 73065?dopt=Abstract [retrieved on 2015-03-05]
- THAKER KUNTAL M ET AL: "Novel markers of cirrhosis and hepatocellular carcinoma in hepatitis C", GASTROENTEROLOGY, vol. 130, no. 4, Suppl. 2, April 2006 (2006-04), page A80, XP002736871, DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 -24, 2006 ISSN: 0016-5085
- WEN WANG: "Detection of Serum Markers of Primary Hepatocellular Carcinoma by ELISA Using Synthetic Polypeptide Antigen", CHINESE JOURNAL OF BIOLOGICALS, vol. 22, no. 4, 1 April 2009 (2009-04-01), pages 399-401, XP055174339,

### SEQUENCE LISTING

<110> Universita' degli Studi della Basilicata
<120> METHOD FOR DIAGNOSING THE RISK OF PRENEOPLASTIC AND NEOPLASTIC LIVER
   DISEASE IN SUBJECTS AFFECTED BY HEPATITIS
<130> 12974PTWO
<150> ITPZ2014A000005
   <151> 2014-06-25
<160> 6
<170> BiSSAP 1.3
<210> 1
   <211> 99
   <212> PRT
   <213> Homo sapiens
<220>
   <223> URG7
<400> 1
   Met Ala Ala Pro Ala Glu Pro Cys Ala Gly Gln Gly Val Trp Asn Gln
   1               5                   10                  15
   Thr Glu Pro Glu Pro Ala Ala Thr Ser Leu Leu Ser Leu Cys Phe Leu
               20                  25                  30
   Arg Thr Ala Gly Val Trp Val Pro Pro Met Tyr Leu Trp Val Leu Gly
           35                  40                  45
   Pro Ile Tyr Leu Leu Phe Ile His His His Gly Arg Gly Tyr Leu Arg
       50                  55                  60
   Met Ser Pro Leu Phe Lys Ala Lys Met Val Ala Ala Ile Pro Gly Ser
   65                  70                  75                  80
   Leu Glu Pro Gly Asn Val Arg Gly Arg Gln Gly Thr Gly Trp Asn Leu
                   85                  90                  95
   Val Lys Ser
<210> 2
   <211> 1503
   <212> PRT
   <213> Homo sapiens
<220>
   <223> MRP6
<400> 2
   Met Ala Ala Pro Ala Glu Pro Cys Ala Gly Gln Gly Val Trp Asn Gln
   1               5                   10                  15
   Thr Glu Pro Glu Pro Ala Ala Thr Ser Leu Leu Ser Leu Cys Phe Leu
               20                  25                  30
   Arg Thr Ala Gly Val Trp Val Pro Pro Met Tyr Leu Trp Val Leu Gly
           35                  40                  45
   Pro Ile Tyr Leu Leu Phe Ile His His His Gly Arg Gly Tyr Leu Trp
       50                  55                  60
   Met Ser Pro Leu Phe Lys Ala Lys Met Val Leu Gly Phe Ala Leu Ile
   65                  70                  75                  80
   Val Leu Cys Thr Ser Ser Val Ala Val Ala Leu Trp Lys Ile Gln Gln
                   85                  90                  95
   Gly Thr Pro Glu Ala Pro Glu Phe Leu Ile His Pro Thr Val Trp Leu
               100                 105                 110
   Thr Thr Met Ser Phe Ala Val Phe Leu Ile His Thr Glu Arg Lys Lys
   115 120 125
   Gly Val Gln Ser Ser Gly Val Leu Phe Gly Tyr Trp Leu Leu Cys Phe
   130 135 140
   Val Leu Pro Ala Thr Asn Ala Ala Gln Gln Ala Ser Gly Ala Gly Phe
   145 150 155 160
   Gln Ser Asp Pro Val Arg His Leu Ser Thr Tyr Leu Cys Leu Ser Leu
   165 170 175
   Val Val Ala Gln Phe Val Leu Ser Cys Leu Ala Asp Gln Pro Pro Phe
   180 185 190
   Phe Pro Glu Asp Pro Gln Gln Ser Asn Pro Cys Pro Glu Thr Gly Ala
   195 200 205
   Ala Phe Pro Ser Lys Ala Thr Phe Trp Trp Val Ser Gly Leu Val Trp
   210 215 220
   Arg Gly Tyr Arg Arg Pro Leu Arg Pro Lys Asp Leu Trp Ser Leu Gly
   225 230 235 240
   Arg Glu Asn Ser Ser Glu Glu Leu Val Ser Arg Leu Glu Lys Glu Trp
   245 250 255
   Met Arg Asn Arg Ser Ala Ala Arg Arg His Asn Lys Ala Ile Ala Phe
   260 265 270
   Lys Arg Lys Gly Gly Ser Gly Met Lys Ala Pro Glu Thr Glu Pro Phe
   275 280 285
   Leu Arg Gln Glu Gly Ser Gln Trp Arg Pro Leu Leu Lys Ala Ile Trp
   290 295 300
   Gln Val Phe His Ser Thr Phe Leu Leu Gly Thr Leu Ser Leu Ile Ile
   305 310 315 320
   Ser Asp Val Phe Arg Phe Thr Val Pro Lys Leu Leu Ser Leu Phe Leu
   325 330 335
   Glu Phe Ile Gly Asp Pro Lys Pro Pro Ala Trp Lys Gly Tyr Leu Leu
   340 345 350
   Ala Val Leu Met Phe Leu Ser Ala Cys Leu Gln Thr Leu Phe Glu Gln
   355 360 365
   Gln Asn Met Tyr Arg Leu Lys Val Leu Gln Met Arg Leu Arg Ser Ala
   370 375 380
   Ile Thr Gly Leu Val Tyr Arg Lys Val Leu Ala Leu Ser Ser Gly Ser
   385 390 395 400
   Arg Lys Ala Ser Ala Val Gly Asp Val Val Asn Leu Val Ser Val Asp
   405 410 415
   Val Gln Arg Leu Thr Glu Ser Val Leu Tyr Leu Asn Gly Leu Trp Leu
   420 425 430
   Pro Leu Val Trp Ile Val Val Cys Phe Val Tyr Leu Trp Gln Leu Leu
   435 440 445
   Gly Pro Ser Ala Leu Thr Ala Ile Ala Val Phe Leu Ser Leu Leu Pro
   450 455 460
   Leu Asn Phe Phe Ile Ser Lys Lys Arg Asn His His Gln Glu Glu Gln
   465 470 475 480
   Met Arg Gln Lys Asp Ser Arg Ala Arg Leu Thr Ser Ser Ile Leu Arg
   485 490 495
   Asn Ser Lys Thr Ile Lys Phe His Gly Trp Glu Gly Ala Phe Leu Asp
   500 505 510
   Arg Val Leu Gly Ile Arg Gly Gln Glu Leu Gly Ala Leu Arg Thr Ser
   515 520 525
   Gly Leu Leu Phe Ser Val Ser Leu Val Ser Phe Gln Val Ser Thr Phe
   530 535 540
   Leu Val Ala Leu Val Val Phe Ala Val His Thr Leu Val Ala Glu Asn
   545 550 555 560
   Ala Met Asn Ala Glu Lys Ala Phe Val Thr Leu Thr Val Leu Asn Ile
   565 570 575
   Leu Asn Lys Ala Gln Ala Phe Leu Pro Phe Ser Ile His Ser Leu Val
   580 585 590
   Gln Ala Arg Val Ser Phe Asp Arg Leu Val Thr Phe Leu Cys Leu Glu
   595 600 605
   Glu Val Asp Pro Gly Val Val Asp Ser Ser Ser Ser Gly Ser Ala Ala
       610                 615                 620
   Gly Lys Asp Cys Ile Thr Ile His Ser Ala Thr Phe Ala Trp Ser Gln
   625                 630                 635                 640
   Glu Ser Pro Pro Cys Leu His Arg Ile Asn Leu Thr Val Pro Gln Gly
                   645                 650                 655
   Cys Leu Leu Ala Val Val Gly Pro Val Gly Ala Gly Lys Ser Ser Leu
               660                 665                 670
   Leu Ser Ala Leu Leu Gly Glu Leu Ser Lys Val Glu Gly Phe Val Ser
           675                 680                 685
   Ile Glu Gly Ala Val Ala Tyr Val Pro Gln Glu Ala Trp Val Gln Asn
       690                 695                 700
   Thr Ser Val Val Glu Asn Val Cys Phe Gly Gln Glu Leu Asp Pro Pro
   705                 710                 715                 720
   Trp Leu Glu Arg Val Leu Glu Ala Cys Ala Leu Gln Pro Asp Val Asp
                   725                 730                 735
   Ser Phe Pro Glu Gly Ile His Thr Ser Ile Gly Glu Gln Gly Met Asn
               740                 745                 750
   Leu Ser Gly Gly Gln Lys Gln Arg Leu Ser Leu Ala Arg Ala Val Tyr
           755                 760                 765
   Arg Lys Ala Ala Val Tyr Leu Leu Asp Asp Pro Leu Ala Ala Leu Asp
       770                 775                 780
   Ala His Val Gly Gln His Val Phe Asn Gln Val Ile Gly Pro Gly Gly
   785                 790                 795                 800
   Leu Leu Gln Gly Thr Thr Arg Ile Leu Val Thr His Ala Leu His Ile
                   805                 810                 815
   Leu Pro Gln Ala Asp Trp Ile Ile Val Leu Ala Asn Gly Ala Ile Ala
               820                 825                 830
   Glu Met Gly Ser Tyr Gln Glu Leu Leu Gln Arg Lys Gly Ala Leu Val
           835                 840                 845
   Cys Leu Leu Asp Gln Ala Arg Gln Pro Gly Asp Arg Gly Glu Gly Glu
       850                 855                 860
   Thr Glu Pro Gly Thr Ser Thr Lys Asp Pro Arg Gly Thr Ser Ala Gly
   865                 870                 875                 880
   Arg Arg Pro Glu Leu Arg Arg Glu Arg Ser Ile Lys Ser Val Pro Glu
                   885                 890                 895
   Lys Asp Arg Thr Thr Ser Glu Ala Gln Thr Glu Val Pro Leu Asp Asp
               900                 905                 910
   Pro Asp Arg Ala Gly Trp Pro Ala Gly Lys Asp Ser Ile Gln Tyr Gly
           915                 920                 925
   Arg Val Lys Ala Thr Val His Leu Ala Tyr Leu Arg Ala Val Gly Thr
       930                 935                 940
   Pro Leu Cys Leu Tyr Ala Leu Phe Leu Phe Leu Cys Gln Gln Val Ala
   945                 950                 955                 960
   Ser Phe Cys Arg Gly Tyr Trp Leu Ser Leu Trp Ala Asp Asp Pro Ala
                   965                 970                 975
   Val Gly Gly Gln Gln Thr Gln Ala Ala Leu Arg Gly Gly Ile Phe Gly
               980                 985                 990
   Leu Leu Gly Cys Leu Gln Ala Ile Gly Leu Phe Ala Ser Met Ala Ala
           995                 1000                1005
   Val Leu Leu Gly Gly Ala Arg Ala Ser Arg Leu Leu Phe Gln Arg Leu
       1010                1015                1020
   Leu Trp Asp Val Val Arg Ser Pro Ile Ser Phe Phe Glu Arg Thr Pro
   1025                1030                1035                1040
   Ile Gly His Leu Leu Asn Arg Phe Ser Lys Glu Thr Asp Thr Val Asp
                   1045                1050                1055
   Val Asp Ile Pro Asp Lys Leu Arg Ser Leu Leu Met Tyr Ala Phe Gly
               1060                1065                1070
   Leu Leu Glu Val Ser Leu Val Val Ala Val Ala Thr Pro Leu Ala Thr
           1075                1080                1085
   Val Ala Ile Leu Pro Leu Phe Leu Leu Tyr Ala Gly Phe Gln Ser Leu
       1090                1095                1100
   Tyr Val Val Ser Ser Cys Gln Leu Arg Arg Leu Glu Ser Ala Ser Tyr
   1105                1110                1115                1120
   Ser Ser Val Cys Ser His Met Ala Glu Thr Phe Gln Gly Ser Thr Val
                   1125                1130                1135
   Val Arg Ala Phe Arg Thr Gln Ala Pro Phe Val Ala Gln Asn Asn Ala
               1140                1145                1150
   Arg Val Asp Glu Ser Gln Arg Ile Ser Phe Pro Arg Leu Val Ala Asp
           1155                1160                1165
   Arg Trp Leu Ala Ala Asn Val Glu Leu Leu Gly Asn Gly Leu Val Phe
       1170                1175                1180
   Ala Ala Ala Thr Cys Ala Val Leu Ser Lys Ala His Leu Ser Ala Gly
   1185                1190                1195                1200
   Leu Val Gly Phe Ser Val Ser Ala Ala Leu Gln Val Thr Gln Thr Leu
                   1205                1210                1215
   Gln Trp Val Val Arg Asn Trp Thr Asp Leu Glu Asn Ser Ile Val Ser
               1220                1225                1230
   Val Glu Arg Met Gln Asp Tyr Ala Trp Thr Pro Lys Glu Ala Pro Trp
           1235                1240                1245
   Arg Leu Pro Thr Cys Ala Ala Gln Pro Pro Trp Pro Gln Gly Gly Gln
       1250                1255                1260
   Ile Glu Phe Arg Asp Phe Gly Leu Arg Tyr Arg Pro Glu Leu Pro Leu
   1265                1270                1275                1280
   Ala Val Gln Gly Val Ser Phe Lys Ile His Ala Gly Glu Lys Val Gly
                   1285                1290                1295
   Ile Val Gly Arg Thr Gly Ala Gly Lys Ser Ser Leu Ala Ser Gly Leu
               1300                1305                1310
   Leu Arg Leu Gln Glu Ala Ala Glu Gly Gly Ile Trp Ile Asp Gly Val
           1315                1320                1325
   Pro Ile Ala His Val Gly Leu His Thr Leu Arg Ser Arg Ile Ser Ile
       1330                1335                1340
   Ile Pro Gln Asp Pro Ile Leu Phe Pro Gly Ser Leu Arg Met Asn Leu
   1345                1350                1355                1360
   Asp Leu Leu Gln Glu His Ser Asp Glu Ala Ile Trp Ala Ala Leu Glu
                   1365                1370                1375
   Thr Val Gln Leu Lys Ala Leu Val Ala Ser Leu Pro Gly Gln Leu Gln
               1380                1385                1390
   Tyr Lys Cys Ala Asp Arg Gly Glu Asp Leu Ser Val Gly Gln Lys Gln
           1395                1400                1405
   Leu Leu Cys Leu Ala Arg Ala Leu Leu Arg Lys Thr Gln Ile Leu Ile
       1410                1415                1420
   Leu Asp Glu Ala Thr Ala Ala Val Asp Pro Gly Thr Glu Leu Gln Met
   1425                1430                1435                1440
   Gln Ala Met Leu Gly Ser Trp Phe Ala Gln Cys Thr Val Leu Leu Ile
                   1445                1450                1455
   Ala His Arg Leu Arg Ser Val Met Asp Cys Ala Arg Val Leu Val Met
               1460                1465                1470
   Asp Lys Gly Gln Val Ala Glu Ser Gly Ser Pro Ala Gln Leu Leu Ala
           1475                1480                1485
   Gln Lys Gly Leu Phe Tyr Arg Leu Ala Gln Glu Ser Gly Leu Val
       1490                1495                1500
<210> 3
   <211> 25
   <212> PRT
   <213> Homo sapiens
<220>
   <223> Peptide75-99 URG7
<400> 3
   Ala Ala Ile Pro Gly Ser Leu Glu Pro Gly Asn Val Arg Gly Arg Gln
   1               5                   10                  15
   Gly Thr Gly Trp Asn Leu Val Lys Ser
               20                  25
<210> 4
   <211> 111
   <212> PRT
   <213> Homo sapiens
<220>
   <223> Polipeptide His-URG7
<400> 4
   Met Arg Gly Ser His His His His His His Gly Ser Met Ala Ala Pro
   1               5                   10                  15
   Ala Glu Pro Cys Ala Gly Gln Gly Val Trp Asn Gln Thr Glu Pro Glu
               20                  25                  30
   Pro Ala Ala Thr Ser Leu Leu Ser Leu Cys Phe Leu Arg Thr Ala Gly
           35                  40                  45
   Val Trp Val Pro Pro Met Tyr Leu Trp Val Leu Gly Pro Ile Tyr Leu
       50                  55                  60
   Leu Phe Ile His His His Gly Arg Gly Tyr Leu Arg Met Ser Pro Leu
   65                  70                  75                  80
   Phe Lys Ala Lys Met Val Ala Ala Ile Pro Gly Ser Leu Glu Pro Gly
                   85                  90                  95
   Asn Val Arg Gly Arg Gln Gly Thr Gly Trp Asn Leu Val Lys Ser
               100                 105                 110
<210> 5
   <211> 28
   <212> DNA
   <213> Homo sapiens
<220>
   <223> Oligonucleotide forward primer 5', 3'
<400> 5
   tcgcggatcc atggccgcgc ctgctgag 28
<210> 6
   <211> 32
   <212> DNA
   <213> Homo sapiens
<220>
   <223> Oligonucleotide reverse primer 5', 3'
<400> 6
   cgaaagcttt caagacttca ccaggttcca gc 32

## Description

### Field of the invention

The invention relates to a method for diagnosing the risk of preneoplastic and neoplastic liver diseases in patients affected by hepatitis virus infection based on the use of the peptide fragment 75-99 of the URG7 protein.

### Background of the invention

It is known that the hepatitis B virus contributes to determining the onset of serious pathological consequences, such as chronic diseases of the liver, cirrhosis and hepatocellular carcinoma, one or more proteins encoded by this virus playing a significant role in hepatocarcinogenesis. In particular, hepatitis and cirrhosis are among the most common risk factors of hepatocellular carcinoma (HCC), which is one of the most frequent cancers at global level and with limited diagnostic options. In fact, presently, the methods most used for diagnosing hepatocellular carcinoma are based on instrumental methods for ultrasound imaging and liver biopsies that detect visible cancers. However, both with the instrumental investigations and with biopsies, it is difficult to distinguish between cirrhotic nodules and a differentiated HCC or dysplastic HCC nodules having a low degree of differentiation. To overcome the limitations of these diagnostic methods, a method based on the dosage of alpha-fetoprotein in the blood has been developed, which finds support in the fact that the diagnosis of hepatocellular carcinoma was associated with a high concentration of this protein in the blood [McMahon BJ, et al. Hepatology, 2000, 32, 842-846; Toyoda H. et al. Clin Gastroenterol Hepatol., 2006, 4, 1170-1176; Thompson Coon J et al. Health Technol Assess., 2007, 11, 1-206; Volk ML et al. Cancer Biomark., 2007, 3, 79-87]. However, a method of diagnosis based on the determination of this protein is strongly limited by its low specificity [Sheman M. et al. J Hepatol., 2001, 34, 603-5; Oka H. et al. Hepatology, 1994, 19, 61-66].

Since the efficacy of the treatment of hepatocellular carcinoma largely depends on an early diagnosis to the onset of the pathological condition, there is a great need to have a method capable of diagnosing any progression toward a hepatocellular carcinoma in the initial stage when it is not yet detectable using instrumental methods for images.

It has now been ascertained that the hepatitis B virus x antigen (HBxAg) is involved in the pathogenetic progression of chronic hepatitis into hepatocellular carcinoma. This antigen is capable of altering the gene expression profile of the host cell [Sui Hie-Won L. Hann, et al. Cancer Res., 2004, 64, 7329-7335]. Of the over-expressed proteins, a particularly important role appears to be played by the URG7 protein, encoded by the URG7 gene (up-regulated gene 7), which appears to play an active role in blocking the apoptotic processes of the infected cells induced by TNFα, interacting with the beta catenin and directly inhibiting caspases 3 and 8 [Pan J. et al. Journal of General Virology, 2007, 88, 3275-3285]. The determination of anti-URG7 antibodies, in addition to anti-Sui1, in the serum of patients affected by hepatitis B long before the diagnosis of HCC, could have important prognostic significance since the appearance thereof appears to be associated with the formation of dysplastic nodules rather than the risk of developing hepatocellular carcinoma (HCC) generally associated with the appearance of anti-URG11, anti-URG4 and anti-S15 antibodies [Hie-Won L. Hann et al. Cancer Res., 2004, 64, 7329-7335].

The URG7 protein consists of 99 amino acids and the gene that encodes it is a 2271-nucleobase gene [Lian Z. et al. Hepatology, 2001, 34, 146-157]. This gene was subsequently identified as the pseudogene 2 of the ABCC6 gene, a gene that encodes a transport protein of 1503 amino acids, MRP6, which mutations are associated with the onset of pseudoxanthoma elasticum, a connective tissue disorder [Li Q. et al. Matrix Biol., 2014, 33C, 23-28]. Studies carried out on the ABCC6 gene have demonstrated the existence of truncated forms of ABCC6 arising from alternative splicing processes [Armentano, M. F. et al. Research Letters in Biochemistry, 2008, doi:10.1155/2008/912478]. It has also been demonstrated that the ABCC6 gene is over-expressed in certain human cancer cell lines [Kool M. et al. Cancer Res., 1999, 59, 175-182] and, recently, in some cancers, such as non-small cell lung cancer and ovarian cancer after chemotherapy treatment. Moreover, its expression is associated with reduced apoptotic activity induced by anticancer drugs [Ikeda R. et al. Int. J. Oncol., 2011, 38, 513-9; Luo H. et al. Cancer Cell Int., 2010, 10, 16]. Ostuni et al., FEBS Letters 587, pp3058-3062, 2013, discloses that antibodies against URG7 are detected in the serum of HBV carriers before the appearance of HCC. Therefore URG7 could be used as a preneoplastic marker to identify HBV carriers at risk of developing HCC and could represent a molecular target for anti-tumor therapies.

Since the amino acid sequence of the URG7 protein is identical in the first 74 residues with the MRP6 protein, it must be established with certainty if the anti-URG7 antibodies found in patients affected by hepatitis B [Hie-Won L. Hann et al. 2004, *ref. cit*.] are due to the over-expression of URG7 or of MRP6. At present, the detection of anti-URG7 antibodies in patients infected with HBV is performed using antigens as the N-terminal sequences12-25 and 56-70 of URG7 [Hie-Won L. Hann et al., 2004, *ref. cit*; US Patent No. 2007/0048808] that coincide with the N-terminal 12-25 and 56-70 sequences of the MRP6 protein.

Although immunochemical methods and kits are widely used to detect early markers of disease their use is often limited by poor specificity. The search for ever-more specific tests is therefore a basic necessity for a correct diagnosis and the present invention allows identification of the presence of anti-URG7 antibodies as a specific preneoplastic marker.

### Summary of the invention

One purpose of the present invention is, therefore, the development of a diagnostic method based on the use of an antigenic peptide that belongs to the URG7 protein and is non superimposable to the sequence of the MRP6 protein, to detect specific anti-URG7 antibodies in the serum of patients affected by chronic hepatitis infection, and by HBV in particular.

In one aspect, the present invention concerns a method for diagnosing the risk of preneoplastic and neoplastic liver diseases in subjects affected by hepatitis virus infection, by hepatitis B in particular, based on detecting specific antibodies against the peptide fragments 75-99 of the URG7 protein consisting of the peptide with the aaipgs lepgnvrgrq gtgwnlvks sequence identified as SEQ ID NO:3, in a biological sample, preferably serum, of these subjects.

Therefore, in a first aspect the object of the invention is a method for diagnosing the risk of preneoplastic and neoplastic liver diseases in subjects affected by hepatitis virus infection comprising at least the step of:
- determining in a biological sample the presence of anti-URG7 antibodies by detection thereof with the peptide of SEQ ID NO:3, wherein the presence of antibodies in the biological sample is indicative of a low risk of progression from chronic hepatitis to preneoplastic and neoplastic liver diseases in subjects affected by viral hepatitis.

In another aspect, the object of the present invention is the use of an immunochemical kit for detecting specific antibodies against the URG7 protein in a biological sample, preferably serum, of patients affected by hepatitis virus infection, in particular HVB, comprising at least: one container with the peptide of SEQ ID NO:3, consisting of the peptide fragment 75-99 of the URG7 protein, one container with an anti-human IgG secondary antibody conjugated with a label for detecting the signal and an instructions for use.

The method and the use of the kit according to the invention allow an early diagnosis to be made, and with non-invasive methods, when the neoplasia is not yet detectable using instrumental images.

### Brief description of the drawings

Figure 1. The figure shows the absorbance values at 450 nm detected by means of indirect ELISA testing of the serum of healthy subjects, in the presence of the antigen of SEQ ID NO:3 (indicated with □ PEP) and of the antigen of SEQ ID NO:4 (indicated with ■ POLYPEP).
Figure 2. The figure shows the absorbance values at 450 nm detected by means of indirect ELISA testing of the serum of patients affected by hepatitis B infection in the presence of the antigen of SEQ ID NO:3 (indicated with □ PEP) and of the antigen of SEQ ID NO:4 (indicated with ■ POLYPEP).

### Detailed description of the invention

### Definitions

Here, "URG7 protein" means human isoform 2, member of the MRP subfamily of the superfamily of ABC transport proteins (ATP-binding cassette) encoded by the URG7 gene [NCBI accession number NC_000016]. The URG7 protein is the protein identified in the database of the National Center for Biotechnology Information with the access number NP_001072996; the sequence is reported in the sequences listing as SEQ ID NO:1.

"Protein MRP6" means the human protein member of the MRP subfamily of the ABC (ATP-binding cassette) transporter protein superfamily involved in multidrug resistance phenomena. The protein is encoded by the ABCC6 [*Homo sapiens* Gene ID 368] gene. In the National Center for Biotechnology Information database it is identified with the GenBank accession number AAC79696; the sequence is reported in the sequences listing as SEQ ID NO:2.

### Description

The present invention concerns a method for evaluating the presence of antibodies against the 75-99 sequence of the URG7 protein in the serum of patients affected by hepatitis virus infection, in particular by hepatitis B, at risk of developing both a less serious pathological form such as dysplastic liver nodules and more serious forms of liver disease such as cirrhosis and hepatocellular carcinoma.

Essentially, the method according to the invention is based on an immunochemical dosage by means of indirect ELISA (Enzyme-Linked Immunosorbent Assay) assay of the anti-URG7 antibodies in serum samples from patients affected by hepatitis. The immunochemical assay can be performed according to the methods known to a person skilled in the art and, in particular, an immunochemical dosage based on the detection of a radiochemical, fluorescent or enzymatic signal by means of spectrophotometric, fluorimetric or chemiluminescent measurements.

This is a versatile and widely used immunological analysis method that allows to detect the presence and dosage of an antigen (direct *ELISA*) or of a specific antibody against an antigen (indirect *ELISA*).

Therefore, in one embodiment of the invention, the method for diagnosing the risk of preneoplastic and neoplastic diseases in subjects affected by hepatitis virus infection, and preferably by hepatitis B, comprises at least the step of determining the presence of anti-URG7 antibodies by detection thereof with the peptide of SEQ ID NO:3 in a serum sample of these subjects.

The risk assessment of a progression of the disease from chronic hepatitis to precancerous or neoplastic diseases in patients affected by chronic viral hepatitis, by hepatitis B in particular, is carried out by comparing the results obtained on the samples of subjects affected by this disease with samples (sera) obtained from healthy subjects. Determinations of the anti-URG7 antibodies on healthy subjects can be carried out autonomously to obtain standard reference values or during the diagnostic test.

In a preferred embodiment the antigen is bound to a support selected from a polystyrene plate, nitrocellulose filter, but chips, microarrays, functionalized resins can be used.

For the purposes of the method according to the invention, it is necessary to immobilize on a plate at least 700 ng, and preferably 1 µg of the peptide 75-99, and at least 50 ng and preferably 200 ng of the His-URG7 polypeptide (SEQ ID NO:4) used as control.

The signal is detected by means of an anti-human IgG secondary antibody conjugated with a marker enzyme, such as for example horseradish peroxidase, alkaline phosphatase, B-galactosidase, glucose oxidase, urease, or derivatized with a fluorophore, such as fluorescein and tetramethylrhodamine for example.

As regards the detection systems, in order to increase sensitivity, reduce the consumption of reagents and sample and reduce the analysis time, biosensors based on advanced technologies of the optical type, such as surface plasmon resonance (SPR), or electrochemical devices such as microelectrodes, can also be used.

Therefore, in a preferred embodiment, the method for diagnosing the risk of preneoplastic and neoplastic diseases in subjects affected by hepatitis virus infection, preferably of type B, provides that the determination of the presence of anti-URG7 antibodies, by means of the detection thereof with the peptide of SEQ ID NO:3, in a biological sample of these subjects comprises the steps of:
- binding the antigen consisting of the peptide of SEQ ID NO:3 to a solid support;
- adding the biological sample, preferably serum, of the subject affected by hepatitis virus infection and reacting with the immobilized antigen;
- adding an anti-human IgG secondary antibody conjugated with a label;
- detecting the signal resulting from the reaction of antigen/specific anti-URG7 antibodies (primary antibodies)/ labelled anti-human IgG secondary antibody;
- comparing the value of the signal detected in the biological sample of the subject affected by hepatitis virus infection against the average value of the signal previously detected in samples, preferably serum, of healthy control subjects.

Optionally, the method can comprise the further step of adding a biological sample (serum) of one or more healthy control subjects.

A value of the signal, substantially absorbance at 450nm, greater than that of the average of the healthy control samples (sera) more than twice the standard deviation, is considered a positive value, indicative of the presence of specific antibodies in assayed serum.

Optionally, as a positive control, the His-URG7 polypeptide (SEQ ID NO:4) can also be immobilized on a similar support. The supports with this polypeptide are then treated like the supports treated with the peptide of SEQ ID NO:3 and the procedure to be followed is entirely similar to what has previously been described for this antigen.

The method for diagnosing the risk of preneoplastic and neoplastic liver diseases in subjects affected by hepatitis virus infection according to the invention can therefore comprise the further steps of:
- binding the antigen consisting of the polypeptide of SEQ ID NO:4 to a solid support;
- adding the biological sample and reacting with the immobilized antigen;
- adding an anti-human IgG secondary antibody conjugated with a label;
- detecting the signal resulting from the reaction of antigen/specific anti-URG7 antibodies (primary antibodies)/ labelled anti-human IgG secondary antibody;
- comparing the value of the signal detected in the biological sample of the subject affected by infection at the signal value detected in a biological sample of a healthy control subject.

The method for diagnosing the risk of preneoplastic and neoplastic diseases in subjects affected by hepatitis virus infection B based on the detection of anti-URG7 antibodies can be achieved with the use of a kit comprising at least: a container containing the antigen consisting of the peptide of SEQ ID NO:3, optionally in combination with a second container containing an anti-human IgG secondary antibody conjugated with a label, and a an information leaflet of the procedure and with an indication of the standard values of these anti-URG7 antibodies in healthy control subjects. Optionally, the kit may further comprise a container with the polypeptide His-URG7 of SEQ ID NO:4 as control. Preferably, both the peptide of SEQ ID NO:3 and the polypeptide of SEQ ID NO:4 are bound to a suitable support for carrying out the analysis.

### EXPERIMENTAL PART

### Example 1. Solid phase synthesis of peptide 75-99 of the URG7 protein (SEQ ID NO:3)

Before proceeding to the synthesis of the peptide 75-99 specific for the URG7 protein, prediction studies for the presence of epitopes were carried out using different software available on the network. The evaluation of the immunogenic potential of an amino acid sequence is carried out by taking various parameters, such as flexibility, accessibility, degree of hydrophilicity and homology with known epitopes, into consideration. The most reliable information was obtained using the *ABCPRED* software, which exploits a database (*Bcipep*) of amino acid sequences recognised as epitopes and capable of triggering an immune response in a host organism and of reacting with the products of this immune response. From the epitope prediction study it has emerged that the non-homologous region of 25 amino acids between the URG7 polypeptide and the MRP6 protein could possess a potential immunogenicity, and could therefore be used for the production of a specific antibody specific for the URG7 polypeptide.

The peptide corresponding to the region 75-99 of the URG7 protein was synthesized using the solid-phase synthesis method with Fmoc strategy on an automatic Pioneer PerSeptive Biosystems synthesizer. Novabiochem amino acids were used with the amino groups protected by the Fmoc (9-flurenyl-methoxycarbonyl) protecting group removed in a basic environment (20% piperidine in dimethylformamide, DMF). Protection of the amino acid amine group is essential during activation of the carboxylic group so that the latter reacts exclusively with the free amino group on the last amino acid residue of the growing peptide chain. An amino acid:functional group of the resin molar ratio equal to 4:1 was used. The resin employed is Fmoc-PAL-PEG-PS (Applied Biosystem), consisting of a copolymer of polystyrene and polyethylene glycol. Binding of the first amino acid to the resin is facilitated by a linker of the PAL-type (Fmoc-aminomethyl-2,5-dimethoxy-phenoxy valeric acid). Assembly of the amino acid chain consists of a series of cyclic deprotection reactions of the amino protector group of the residue bound to the growth and condensation peptide between the amino group and the activated carboxyl function of a new residue, with formation of a peptide bond. Amino acids, beyond the amino terminus and the carboxylic acid terminus, can contain further reactive groups in the side chain: a protection strategy of the side chains with groups that prove stable will therefore be necessary. The reagent employed for activation of the carboxylic function was PyBOP (benzotriazole-1-yl-oxytripyrrolidinophosphonium hexafluorophosphate) (0.5 M in DMF) in the presence of DIPEA (N,N-diisopropylethylamine) (1M in DMF).

On completion of the synthesis, the peptide was detached from the resin and the protector groups of the amino acid side chains were simultaneously removed using crystalline phenol 0.75 g, ethanedithiol 0.25 ml, thioanisole 0.25 ml, deionized water 0.5 ml, trifluoroacetic acid 10 ml (TFA).

After 3 hours of reacting at room temperature, the suspension was filtered on a semipermeable membrane, adding 2 ml of TFA and 4 ml of dichloromethane (DCM). The filtrate containing the peptide was concentrated in a rotavapor at a temperature of about 30°C, and the peptide was precipitated with ethyl ether, isolated by centrifugation solubilised in H₂O/TFA 0.1% and CH₃CN/TFA O.1% in a ratio of 1:1 and, lastly, lyophilized. The peptide was then purified by reversed-phase high performance liquid chromatography (RP-HPLC) on a C18 column (Jupiter 5 µm 300 Å, 250X10.00 mm, Phenomenex) and with a gradient of the mobile phase, consisting of Acetonitrile + 0.1% TFA, water+TFA ranging from 5% to 60% acetonitrile in 30 minutes, maintaining the concentration at 60% for 10 minutes and returning to 5% acetonitrile in 5 minutes. The flow rate is equal to 3 ml/min. The fraction relating to the peptide was collected, freeze-dried and analysed by electrospray mass spectrometry.

### Example 2. Preparation of the His-URG7 polypeptide (SEQ ID NO:4) by recombinant DNA technology.

The His-URG7 polypeptide was amplified by PCR from cDNA of hepatoma HepG2 cells using as forward primer 5'-TCGCGGATCCATGGCCGCGCCTGCTGAG-3' (SEQ ID NO:5) and as reverse primer 5'-CGAAAGCTTTCAAGACTTCACCAGGTTCCAGC-3' (SEQ ID NO:6). The gene was then cloned in the expression vector pQE30, transformed into Rosetta pLYS-S *E.coli* strain (Novagen), and expressed as His-tagged polypeptide to the N-terminal (His-URG7). Purification was carried out by means of RP-HPLC [Ostuni A. et al. Prot. Pept. Lett., 2014, 21 (5), 413-8].

### Example 3. Production of the specific polyclonal antibody for the peptide 75-99 of SEQ ID NO:3

The peptide 75-99 URG7 with an N-terminal cysteine residue (Cys-URG7) conjugated with the protein carrier, KLH (Keyhole Limpet Hemocyanin), and mixed in a 1:1 ratio with alum (aluminium hydroxide) as adjuvant was used to immunize white rabbits (*New Zealand*) according to the standard procedure implemented by the company PRIMM srl (Milan). Each rabbit was subcutaneously injected with a dose of immunogen equal to 250 µg. The following challenges with 200 µg of conjugate were carried out: a first follow-up at a distance of three weeks and three other follow-ups at a distance of one week from the another.

The serum (S) was provided as a such and also purified by means of affinity chromatography (A).

### Example 4. Evaluation of the antigen immobilization methodology and of the immunochemical reactivity thereof by means of the ELISA test

In order to determine the optimal conditions to be used in the assay to detect any specific anti-URG7 antibodies in the serum of patients, preliminary indirect ELISA experiments were carried out, fixing onto a plate both the peptide 75-99 (SEQ ID NO:3) and the His-URG7 polypeptide (SEQ ID NO:4) at different concentrations and using different dilutions of both the immune rabbit serum (S) and the purified anti-URG7 IgG antibodies (A). Different experimental conditions were considered in order to optimize the binding of the antigen and solubilisation of the peptide 75-99 and the His-URG7 polypeptide in order to keep them in the immunochemically active form.

The optimal experimental procedure is described below:
1. add of 100 µL of glutaraldehyde at 5% onto a multiwell plate (Nunc Brand Products-Polysorp) and incubate for 1 hour at room temperature;
2. gently empty the wells and perform 5 washes with 1X PBS (200 µl/well);
3. in accordance with the plate diagram add 100 µl of antigen: use a 10 µg/ml solution of the peptide 75-99 in PBS 1X and use a 2 µg/ml solution of His-URG7 in 70% TFE;
4. incubate the plate at 4 °C overnight;
5. gently empty the wells and perform 3 washes with PBS 1X (200 µL per well);
6. add 200 µl of a solution of 5% powdered skimmed milk in PBS 1X to all the wells in which the assay is to be performed and incubate for 2 hours at room temperature;
7. gently empty the wells and perform 3 washes with PBS 1X (200 µl per well);
8. in accordance with the plate diagram add 50 µl of a solution of serum and purified antibody, suitably diluted in PBS 1% powdered skimmed milk and incubate the plate for 1 hour at room temperature;
9. gently empty the wells and perform 8 washes with PBST 1X (Tween 20 at 0.05% (200 µL per well);
10.in accordance with the plate diagram add 50 µl of a solution of secondary antibody (anti-rabbit IgG-HRP Sigma) diluted1:5,000 in PBS 1% powdered skimmed milk;
11.incubate the plate for 1 hour at room temperature;
12.gently empty the wells and perform 8 washes with PBST 1X (Tween 20 at 0.05% (200 µL per well);
13.add 50 µl of a solution of OPD (o-phenylenediamine dihydrochloride) in citrate phosphate buffer pH 9.6 (0.8 mg of OPD in 1 ml of citrate phosphate buffer) to each well;
14.leave the plate in the dark for 30 minutes and perform a spectrophotometer reader (λ=450nm).

The amount of antigen (1 µg for the peptide 75-99 and 200 ng for His-URG7) was maintained fixed and the dilutions 1:100, 1:1000, 1:3000, 1:7000, 1:20000 and 1:50000 for the serum (S) and the concentrations 2 µg/ml, 1 µg/ml, 0.5 µg/ml, 0.1 µg/ml, 0.05 µg/ml and 0.01 µg/ml for the purified IgG antibodies (A), were tested. The absorbance values at 450 nm in the indirect ELISA assay using the immune rabbit serum (S) and the purified anti-URG7 antibodies (A) are indicated in TABLE I (A and B, respectively):

**TABLE I A**

| **ANTIGENS** | **DILUTION OF RABBIT IMMUNE SERUM** | | | | | | Negative control |
|---|---|---|---|---|---|---|---|
| | 1/100 | 1/1000 | 1/3000 | 1/7000 | 1/20000 | 1/50000 | |
| *peptide 75-99* | 1.368 | 1.110 | 0.663 | 0.287 | 0.103 | 0.057 | 0.221 |
| *His-URG7* | 1.389 | 1.369 | 0.987 | 0.505 | 0.251 | 0.103 | 0.246 |

**TABLE I B**

| **ANTIGENS** | **PURIFIED ANTIBODY CONCENTRATIONS** | | | | | | Negative control |
|---|---|---|---|---|---|---|---|
| | 2 µg/µL | 1 µg/µL | 0.5 µg/µL | 0.1 µg/µL | 0.05µg/µL | 0.01 µg/µL | |
| *peptide 75-99* | 1.153 | 0.842 | 0.448 | 0.129 | 0.060 | 0.063 | 0.221 |
| *His-URG7* | 1.376 | 1.203 | 0.681 | 0.270 | 0.143 | 0.002 | 0.246 |

From analysis of the data obtained it has been possible to establish that the minimum amount of immune rabbit serum and purified antibody used, if the peptide 75-99 (1 µg) or His-URG7 (200 ng) is immobilized on the plate, is as reported in TABLE II:

**TABLE II**

| | peptide 75-99 | His-URG7 |
|---|---|---|
| IMMUNE SERUM | 1/7000 | 1/20000 |
| Purified antibodies | 0.5 µg/µL | 0.1 µg/µL |

While keeping constant the dilution of the serum (1:7000) and of the purified anti-URG7 antibodies (0.5 µg/ml), various amounts (from 1 µg to 50 ng) of both the peptide 75-99 (Table III A) and of the His-URG7 polypeptide (TABLE III B) were immobilized on a plate in order to determine the minimum optimal amount of antigen for detecting the formation of the specific antigen and antibody complex.

The absorbance values recorded in the ELISA assays carried out are as follow:

**TABLE III A**

| **PRIMARY ANTIBODY** | ***peptide 75-99*** | | | | | | Negative control: |
|---|---|---|---|---|---|---|---|
| | 1 *µg* | 700 ng | 400 ng | 200 ng | 100 ng | 50 ng | |
| IMMUNE SERUM | 0.212 | 0.106 | 0.088 | 0.022 | 0.012 | 0.004 | 0.210 |
| *Purified antibodies* | 0.444 | 0.178 | 0.077 | 0.045 | 0.011 | 0.003 | 0.246 |

**TABLE III B**

| **PRIMARY ANTIBODY** | **His-*URG7*** | | | | | | Negative control |
|---|---|---|---|---|---|---|---|
| | 1 *µg* | 700 ng | 400 ng | 200 ng | 100 ng | 50 ng | |
| *IMMUNE SERUM* | 0.641 | 0.614 | 0.578 | 0.554 | 0.460 | 0.355 | 0.210 |
| *Purified antibodies* | 1.165 | 1.100 | 1.033 | 0.965 | 0.799 | 0.677 | 0.246 |

Analysis of the data obtained has made it possible to determine that plate immobilization of 1 µg of peptide 75-99 and at least 50 ng of the His-URG7 polypeptide is necessary.

### Example 5. Evaluation of the presence of specific anti-URG7 antibodies in the sera of healthy patients affected by hepatitis B by means of indirect ELISA

After optimizing the binding conditions of the two antigens (plate activated with glutaraldehyde and a minimum amount of the antigen to be depositing), stability over time was evaluated (at 4 °C and for at least 3 months). In collaboration with IRCSS C.R.O.B. of Rionero, 18 sera from healthy subjects and 30 sera from patients affected by HBV infection (supplied by operational Extended Care Unit of San Francesco Hospital in Venosa) were assayed.

The ELISA protocol that was followed is the same as the one reported in example 4, but with the following variants:
- point 3: use a solution of the peptide 75-99 10 µg/ml in 1X PBS (1 µg per well) and a solution of His-URG7 in 70% TFE 2 µg/ml (200 ng/well);
- point 8: add 100 µl of the patient's serum diluted 1:50 in PBS 1% powdered milk;
- point 10: use, as secondary antibody, rabbit anti-human IgG-HRP (Santa Cruz, sc-2769).

All the tests were carried out in triplicate and the appropriate negative controls (absorbance evaluation in wells with the patient's serum only and antibody II but without an antigen) were also carried out.

### Results

So as not to influence evaluation of the results, a "blind" study was carried out, i.e. the investigator was not aware of the clinical status of the patient whose serum was being assayed. The result of the immunochemical dosage and the clinical data was then associated.

The sera of 12 healthy subjects (8 females with average age of 39±9; 4 males with average age of 43± 4) and 30 sera of patients affected by HBV infection (3 females 3 with average age of 72±16; 27 males with average age of 51±11) were examined. All the patients examined proved HbsAg positive, anti-HDV negative, with normal alpha-fetoprotein and only 14 under conventional pharmacological regimen (with interferon and/or nucleotide/nucleosides analogues).

In the case of active carriers (19 in the study) the values of HBV-DNA at the onset ranged from a minimum of 299 IU/ml to a maximum of approximately 160,000000 IU/ml. In most cases, after pharmacological treatment, HBV-DNA converted from positive to negative or at most remained at the values of 396 IU/ml (7,600,000 at onset). The sera evaluated were all obtained from patients HBV-DNA negative patients.

In 6 HBV-DNA positive patients, a diagnosis of cirrhosis was made, while only 1 turned out to be affected by hepatocellular carcinoma due to HBV infection and 1 patient was affected by hepatocellular carcinoma from HCV (with cirrhosis and died before commencing a therapeutic programme).

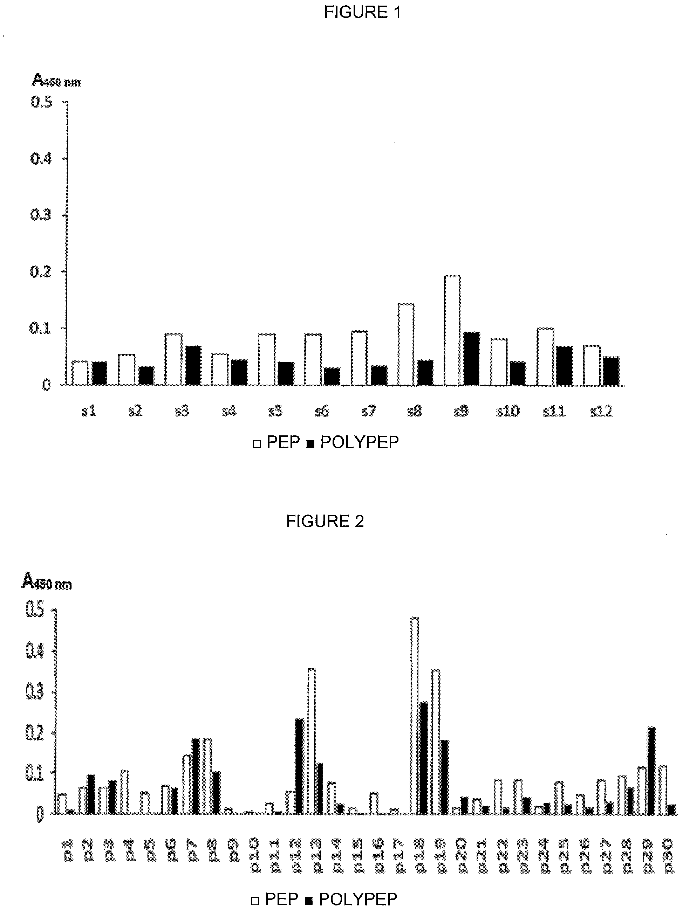

Under optimised experimental conditions, an absorbance value greater than the average absorbance value of the healthy control serum (FIGURE 1) plus twice the standard deviation was considered a positive value, indicative of the presence of specific antibodies in the serum assayed. From the results obtained, it is observed that of the 5 sera positive for the His-URG7 polypeptide (patients 7, 12, 18, 19, 29), only 2 were also positive for the peptide 75-99 (patients 18 and 19), while the serum of patient 13 is only positive for the peptide 75-99 (FIGURE 2).

For two of these patients (patients 13 and 19), neither cirrhosis or HCC was diagnosed, despite the high levels of HBV-DNA at the onset. In patient 18, with a high level of infection at the onset, there is cirrhosis but no progression towards HCC in a year of treatment with Baraclude®. Only one patient with a high level of infection at diagnosis (patient 8), manifested cirrhosis that rapidly evolved into HCC and the anti-URG7 antibody levels for the His-URG7 polypeptide were found to be no different from those of the healthy subjects and at the limit of positivity for the peptide 75-99.

## Claims

1. A method for diagnosis of the risk of preneoplastic and neoplastic liver diseases in subjects affected by viral hepatitis comprising at least the step of:
- determining in a biological sample the presence of anti-URG7 antibodies by detection thereof with the peptide of SEQ ID NO:3 consisting of the peptide fragment 75-99 of the URG7 protein, wherein the presence of the anti-URG7 antibodies in the biological sample is indicative of a low risk of progression from chronic hepatitis to preneoplastic and neoplastic liver diseases in said subjects.

2. The method for diagnosis of the risk of preneoplastic and neoplastic liver diseases in subjects affected by viral hepatitis according to claim 1, wherein the presence of the anti-URG7 antibodies in the assayed sample is evaluated with reference to a signal value resulting from the detection thereof higher than the average signal values obtained on biological samples of healthy subjects plus twice the standard deviation.

3. The method for diagnosis of the risk of preneoplastic and neoplastic liver diseases in subjects affected by viral hepatitis according to claim 1, wherein the step of determining the presence of the anti-URG7 antibodies is carried out by means of an ELISA assay.

4. The method for diagnosis of the risk of preneoplastic and neoplastic liver diseases in subjects affected by viral hepatitis according to claim 1, wherein the biological sample is serum.

5. The method for diagnosis of the risk of preneoplastic and neoplastic liver diseases in subjects affected by viral hepatitis according to one of the claims 1 to 4, comprising the steps of:
- binding the antigen consisting of the peptide of SEQ ID NO:3 to a solid support;
- adding a biological sample of a subject affected by viral hepatitis and reacting with the immobilized antigen;
- adding an anti-human IgG secondary antibody conjugated with a label;
- detecting the signal resulting from the reaction of antigen/specific anti-URG7 antibodies (primary antibodies)/labeled anti-human IgG secondary antibody;
- comparing the value of the signal detected in the biological sample of the subject affected by viral hepatitis with the average signal values obtained on biological samples of healthy subjects.

6. The method for diagnosis of the risk of preneoplastic and neoplastic liver diseases in subjects affected by viral hepatitis according to one of claims 1 to 5, wherein the polypeptide of SEQ ID NO:4 consisting of the His-URG7 polypeptide is used as a positive control.

7. The method for diagnosis of the risk of preneoplastic and neoplastic liver diseases in subjects affected by viral hepatitis according to claim 6, comprising the further steps of:
- binding the antigen consisting of the peptide of SEQ ID NO:4 to a solid support;
- adding a biological sample of a subject affected by viral hepatitis and reacting with the immobilized antigen;
- adding an anti-human IgG secondary antibody conjugated with a label;
- detecting the signal resulting from the reaction of antigen/specific anti-URG7 antibodies (primary antibodies)/labeled anti-human IgG secondary antibody;
- comparing the value of the signal detected in the biological sample of the subject affected by viral hepatitis with the average signal values obtained on biological samples of healthy subjects.

8. The method for diagnosis of the risk of preneoplastic and neoplastic liver diseases in subjects affected by viral hepatitis according to claim 1, wherein the hepatitis is due to the hepatitis B virus.

9. The method for diagnosis of the risk of preneoplastic and neoplastic liver diseases in subjects affected by viral hepatitis according to claim 1, wherein the preneoplastic and neoplastic liver diseases are cirrhosis, hepatocellular carcinoma.

10. Use of a kit for detection of anti URG7 antibodies in a biological sample according to the methods of any of claims 1-9, preferably serum, of a subject affected by viral hepatitis, wherein said kit comprises at least:
- a container with the peptide of SEQ ID NO:3, consisting of the peptide fragment 75-99 of the URG7 protein;
- a further container with an anti-human IgG secondary antibody conjugated with a label for detecting the signal; and
- an information leaflet with instructions for carrying out the method according to claims 1-9.

11. The use of a kit for detection of antibodies anti URG7 according to claim 10, comprising a further container with the polypeptide of SEQ ID NO:4 consisting of the His-URG7 polypeptide.

12. The use of a kit for detection of antibodies anti URG7 protein according to claims 10 or 11, wherein the peptide of SEQ ID NO:3 and the polypeptide of SEQ ID NO:4 are bound to a support.

## Patentansprüche

1. Verfahren zur Diagnose des Risikos an präneoplastischen und neoplastischen Lebererkrankungen bei Personen, die von viraler Hepatitis betroffen sind, aufweisend zumindest den Schritt:
- Nachweis der Anwesenheit von Anti-URG7 Antikörpern in einer biologischen Probe durch Detektion desselben mit dem Peptid aus SEQ ID NO:3, bestehend aus dem Peptid-Fragment 75-99 des URG7-Proteins, wobei die Anwesenheit der Anti-URG7 Antikörper in der biologischen Probe auf ein geringes Risiko der Progredienz von chronischer Hepatitis hin zu präneoplastischen und neoplastischen Lebererkrankungen bei den Personen hinweist.

2. Verfahren zur Diagnose des Risikos an präneoplastischen und neoplastischen Lebererkrankungen bei Personen, die von viraler Hepatitis betroffen sind, gemäß Anspruch 1, wobei die Anwesenheit der Anti-URG7 Antikörper in der untersuchten Probe in Bezug auf einen Signalwert evaluiert wird, der höher liegt als der Durchschnitt aus Detektion desselben in biologischen Proben von gesunden Personen plus der doppelten Standardabweichung.

3. Verfahren zur Diagnose des Risikos an präneoplastischen und neoplastischen Lebererkrankungen bei Personen, die von viraler Hepatitis betroffen sind, gemäß Anspruch 1, wobei der Schritt des Nachweises der Anwesenheit von Anti-URG7 Antikörpern mittels einer ELISA-Untersuchung durchgeführt wird.

4. Verfahren zur Diagnose des Risikos an präneoplastischen und neoplastischen Lebererkrankungen bei Personen, die von viraler Hepatitis betroffen sind, gemäß Anspruch 1, wobei die biologische Probe Serum ist.

5. Verfahren zur Diagnose des Risikos an präneoplastischen und neoplastischen Lebererkrankungen bei Personen, die von viraler Hepatitis betroffen sind, gemäß einem der Ansprüche 1 bis 4, aufweisend die folgenden Schritte:
- Binden des Antigens, bestehend aus dem Peptid der SEQ ID NO:3 an einen festen Träger;
- Hinzufügen einer biologischen Probe einer Person, die von viraler Hepatitis betroffen ist, und reagieren lassen mit dem immobilisierten Antigen;
- Hinzufügen eines Anti-Human-IgG-Sekundär-Antikörpers, der mit einer Kennzeichnung konjugiert ist;
- Detektieren des Signals, das aus der Reaktion von Antigen / spezifischen Anti-URG7 Antikörpern (primären Antikörpern) / gekennzeichneter Anti-Human-IgG-Sekundär-Antikörper resultiert;
- Vergleichen des Signalwerts aus der biologischen Probe der Person, die von viraler Hepatitis betroffen ist, mit dem Durchschnitts-Signalwert aus biologischen Proben von gesunden Personen.

6. Verfahren zur Diagnose des Risikos an präneoplastischen und neoplastischen Lebererkrankungen bei Personen, die von viraler Hepatitis betroffen sind, gemäß einem der Ansprüche 1 bis 5, wobei das Polypeptid aus SEQ ID NO:4, bestehend aus dem His-URG7 Polypeptid als Positivkontrolle verwendet wird.

7. Verfahren zur Diagnose des Risikos an präneoplastischen und neoplastischen Lebererkrankungen bei Personen, die von viraler Hepatitis betroffen sind, gemäß Anspruch 6, weiter aufweisend folgende Schritte:
- Binden des Antigens, bestehend aus dem Peptid aus SEQ ID NO:4, an einen festen Träger;
- Hinzufügen einer biologischen Probe einer Person, die von viraler Hepatitis betroffen ist, und reagieren lassen mit dem immobilisierten Antigen;
- Hinzufügen eines Anti-Human-IgG-Sekundär-Antikörpers, der mit einer Kennzeichnung konjugiert ist;
- Detektieren des Signals, das aus der Reaktion von Antigen / spezifischen Anti-URG7 Antikörpern (primären Antikörpern) / gekennzeichneter Anti-Human-IgG-Sekundär-Antikörper resultiert;
- Vergleichen des Signalwerts aus der biologischen Probe der Person, die von viraler Hepatitis betroffen ist, mit dem Durchschnitts-Signalwert aus biologischen Proben von gesunden Personen.

8. Verfahren zur Diagnose des Risikos an präneoplastischen und neoplastischen Lebererkrankungen bei Personen, die von viraler Hepatitis betroffen sind, gemäß Anspruch 1, wobei die Hepatitis durch das Hepatitis-B-Virus verursacht ist.

9. Verfahren zur Diagnose des Risikos an präneoplastischen und neoplastischen Lebererkrankungen bei Personen, die von viraler Hepatitis betroffen sind, gemäß Anspruch 1, wobei die präneoplastischen und neoplastischen Lebererkrankungen Zirrhose und hepatozelluläres Karzinom sind.

10. Verwendung eines Sets zur Detektion von Anti-URG7 Antikörpern in einer biologischen Probe gemäß einem der Ansprüche 1 bis 9, vorzugsweise Serum, von einer Person, die von viraler Hepatitis betroffen ist, wobei das Set zumindest aufweist:
- einen Behälter mit dem Peptid aus SEQ ID NO:3, bestehend aus dem Peptid-Fragment 75-99 des URG7-Proteins;
- einen weiteren Behälter mit einem Anti-Human-IgG-Sekundär-Antikörper, der mit einer Kennzeichnung konjugiert ist;
- ein Informationsblatt mit Anleitungen zur Durchführung des Verfahrens gemäß den Ansprüchen 1 bis 9.

11. Verwendung eines Sets zur Detektion von Anti-URG7 Antikörpern gemäß Anspruch 10, das einen weiteren Behälter mit dem Polypeptid aus SEQ ID NO:4, bestehend aus dem His-URG7 Polypeptid, aufweist.

12. Verwendung eines Sets zur Detektion von Anti-URG7 Antikörpern gemäß den Ansprüchen 10 oder 11, wobei das Peptid aus SEQ ID NO:3 und das Polypeptid aus SEQ ID NO:4 an einen Träger gebunden sind.

## Revendications

1. Procédé pour diagnostiquer le risque de maladies hépatiques prénéoplasiques et néoplasiques chez des sujets affectés par une hépatite virale comprenant au moins l'étape de :
- détermination dans un échantillon biologique de la présence d'anticorps anti-URG7 par la détection de ceux-ci avec le peptide de SEQ ID NO : 3 consistant en le fragment peptidique 75-99 de la protéine URG7, dans lequel la présence des anticorps anti-URG7 dans l'échantillon biologique indique un faible risque d'évolution d'une hépatite chronique vers des maladies hépatiques prénéoplasiques et néoplasiques chez lesdits sujets.

2. Procédé pour diagnostiquer le risque de maladies hépatiques prénéoplasiques et néoplasiques chez des sujets affectés par une hépatite virale selon la revendication 1, dans lequel la présence des anticorps anti-URG7 dans l'échantillon testé est évaluée en se référant à une valeur de signal résultant de la détection de ceux-ci supérieure aux valeurs de signal moyennes obtenues avec des échantillons biologiques de sujets en bonne santé plus deux fois l'écart-type.

3. Procédé pour diagnostiquer le risque de maladies hépatiques prénéoplasiques et néoplasiques chez des sujets affectés par une hépatite virale selon la revendication 1, dans lequel l'étape de détermination de la présence des anticorps anti-URG7 est réalisée au moyen d'une analyse ELISA.

4. Procédé pour diagnostiquer le risque de maladies hépatiques prénéoplasiques et néoplasiques chez des sujets affectés par une hépatite virale selon la revendication 1, dans lequel l'échantillon biologique est du sérum.

5. Procédé pour diagnostiquer le risque de maladies hépatiques prénéoplasiques et néoplasiques chez des sujets affectés par une hépatite virale selon l'une quelconque des revendications 1 à 4, comprenant les étapes de :
- liaison de l'antigène consistant en le peptide SEQ ID NO : 3 à un support solide ;
- ajout d'un échantillon biologique d'un sujet affecté par une hépatite virale et mise en réaction avec l'antigène immobilisé ;
- ajout d'un anticorps secondaire anti-IgG humaine conjugué à un marqueur ;
- détection du signal résultant de la réaction de l'antigène/anticorps anti-URG7 spécifiques (anticorps primaires)/anticorps secondaire anti-IgG humaine marqué ;
- comparaison de la valeur du signal détecté dans l'échantillon biologique du sujet affecté par une hépatite virale aux valeurs de signal moyennes obtenues avec des échantillons biologiques de sujets en bonne santé.

6. Procédé pour diagnostiquer le risque de maladies hépatiques prénéoplasiques et néoplasiques chez des sujets affectés par une hépatite virale selon l'une quelconque des revendications 1 à 5, dans lequel le polypeptide de SEQ ID NO : 4 consistant en le polypeptide His-URG7 est utilisé comme témoin positif.

7. Procédé pour diagnostiquer le risque de maladies hépatiques prénéoplasiques et néoplasiques chez des sujets affectés par une hépatite virale selon la revendication 6, comprenant les étapes supplémentaires de :
- liaison de l'antigène consistant en le peptide SEQ ID NO : 4 à un support solide ;
- ajout d'un échantillon biologique d'un sujet affecté par une hépatite virale et mise en réaction avec l'antigène immobilisé ;
- ajout d'un anticorps secondaire anti-IgG humaine conjugué à un marqueur ;
- détection du signal résultant de la réaction de l'antigène/anticorps anti-URG7 spécifiques (anticorps primaires)/anticorps secondaire anti-IgG humaine marqué ;
- comparaison de la valeur du signal détecté dans l'échantillon biologique du sujet affecté par une hépatite virale aux valeurs de signal moyennes obtenues avec des échantillons biologiques de sujets en bonne santé.

8. Procédé pour diagnostiquer le risque de maladies hépatiques prénéoplasiques et néoplasiques chez des sujets affectés par une hépatite virale selon la revendication 1, dans lequel l'hépatite est due au virus de l'hépatite B.

9. Procédé pour diagnostiquer le risque de maladies hépatiques prénéoplasiques et néoplasiques chez des sujets affectés par une hépatite virale selon la revendication 1, dans lequel les maladies hépatiques prénéoplasiques et néoplasiques sont une cirrhose, un carcinome hépatocellulaire.

10. Utilisation d'un kit pour la détection d'anticorps anti-URG7 dans un échantillon biologique selon les procédés de l'une quelconque des revendications 1 à 9, de préférence du sérum, d'un sujet affecté par une hépatite virale, dans laquelle ledit kit comprend au moins :
- un récipient avec le peptide de SEQ ID NO : 3, consistant en le fragment peptidique 75-99 de la protéine URG7 ;
- un récipient supplémentaire avec un anticorps secondaire anti-IgG humaine conjugué à un marqueur pour détecter le signal ; et
- une notice d'information avec des instructions pour mettre en oeuvre le procédé selon les revendications 1 à 9.

11. Utilisation d'un kit pour la détection d'anticorps anti-URG7 selon la revendication 10, comprenant un récipient supplémentaire avec le polypeptide de SEQ ID NO : 4 consistant en le polypeptide His-URG7.

12. Utilisation d'un kit pour la détection d'anticorps anti-protéine URG7 selon la revendication 10 ou 11, dans laquelle le peptide de SEQ ID NO : 3 et le polypeptide de SEQ ID NO : 4 sont liés à un support.
